# EUROPEAN PATENT APPLICATION

(11) **EP 1 798 504 A1**
(43) Date of publication of application: **20.06.2007**
(21) Application number: 05112378.4
(22) Date of filing: 19.12.2005
(51) Int. Cl.: F26B 5/06, A61K 9/19

(54) **Method of making dried particles**

(71) Applicant: Koninklijke Philips Electronics N.V., 5621 BA Eindhoven (NL)
(72) Inventor: The designation of the inventor has not yet been filed
(74) Representative: Schmitz, Herman Jan Renier

(57) **Abstract**

A method is provided that prepares resuspendable particles of a compound. The particles are prepared by drying a liquid that comprises the compound. The particles are dried in such a way that they do not aggregate on redispersion. The particles that are thus obtained are especially suitable for use in biosensors, as contrast agent or as part of a pharmaceutical composition.

## Description

### Field of the invention

The invention relates to a method for making a dry solid of particles of a compound, which method includes a step of drying a liquid composition that comprises the compound. The invention further relates to the particles and their use in a device, contrast agent, or pharmaceutical composition.

### Background to the invention

Biosensors are used to detect the presence of specific biomolecules in a sample that is suspected of containing these biomolecules. Biosensors may contain a dried solid e.g. a detectable label, which is suspended in a sample liquid during the assay that is performed in the sensor.

Dried compositions may further be applied as starting compounds for contrast agents. These are generally resuspended before use.

Ideally this dried solid is easily redispersed in a sample liquid such as blood or urine or a carrier composition such as a physiological salt solution. However it has been found that dried particles may aggregate on resuspension thus preventing the formation of a homogeneous particle suspension. This drawback is especially encountered for particles in colloidal solutions or suspensions.

Particles in colloidal solutions are stabilized by several different mechanisms such as electrostatic repulsion or steric interactions to prevent aggregation due to attractive van der Waals forces. Upon drying a colloidal suspension, the particles often aggregate and form clusters. Once in contact, strong van der Waals forces may prevent a resuspension of these colloidal particles. As a concequence, a considerable amount of aggregates is obtained, which can disturb applications, where a homogeneous particle suspension is desired.

Usually, a solution containing many (order of 10¹²-10¹⁴ particles per liter) particles is dried at once, where a contact of particles upon drying can not be prevented. We have found this often leads to aggregate formation during drying and may prevent the formation of a homogeneous suspension on redispersion with a sample liquid.

US-A-2004/0043042 discloses microscale lyophilization techniques for the stabilization and drying of molecules. The drying method that is described includes a) providing a liquid comprising an agent of interest dissolved or dispersed in a volatile liquid medium, (b) depositing a microquantity of between 1 nL to 10 µL of the liquid onto a preselected site of a substrate, (c) drying the microquantity by volatising the liquid medium to produce a solid, dry form of the agent of interest. Optionally this method is a lyophilization method including freezing the microquantity after step (b) and before step (c).

However, it is has been found that this still leads to aggregation of particles on drying which makes it difficult to redisperse them in a monodisperse way.

It is an object of the invention to provide dried particles that easily redisperse in a liquid, without the formation of aggregates.

### Summary of the invention

It has surprisingly been found that the drying of small droplets of liquid that contain on average about one particle per droplet, leads to "single" particles that can easily be redispersed to form a monodisperse liquid suspension.

Therefore in a first aspect the invention relates to a method for making a dry solid of particles of a compound comprising the steps of
a) providing a volatile liquid comprising the compound dissolved or dispersed therein,
b) depositing a quantity of from about 0.1 picoliter to about 1000 nanoliter of the liquid onto a substrate
c) drying the composition to produce a solid form of the compound,
wherein the concentration of the compound in the liquid that is deposited is such that the dried compound is in the form of separate particles.

In a further aspect the invention relates to dried particles that are obtainable by this method.

In another aspect the invention relates to a pharmaceutical composition comprising these dried particles.

In a further aspect the invention relates to a medical device comprising the dried particles and to a method wherein this device is used.

In a further aspect the invention relates to a contrast agent or precursor thereof comprising the dried particles.

### Detailed description of the invention

The invention is especially suitable for the drying of colloidal particles of a compound in a liquid medium, a colloidal solution.

By a colloidal solution one understands a suspension of solid particles that is prevented from fast aggregation by a third or more component(s).

Examples of colloidal solutions are suspensions of latex (magnetic) particles in an aqueous liquid, of iron oxide or other magnetic particles in aqueous liquid, of silver or gold particles in aqueous media, and water-stabilized quantum dots.

The compound that is dried in the form of particles is preferably selected from the group comprising biomolecules, magnetic particles, fluorescent particles, optically active particles such as quantum dots, latex particles, polymer particles and polymer capsules, gold or silver particles, and Gd-containing nanoparticles.

The invention relates to a method for making dry solid particles that are especially suitable for use in a biosensor.

In the context of the invention, the particles generally have an average size (largest diameter) of from 5 to 5000 nm, more preferred from 30 to 500 nm, even more preferred from 80 to 400 nm, even more preferred from 100 to 300 nm.

The particles that are suitable for use in the current invention preferably comprise a core and a shell. The shell generally serves to increase the stability of the particles. Optionally the particles are dispersed in an organic matrix.

In a preferred embodiment, the particles comprise a ligand that is suitable for binding the particle to a target composition.

The compound that is to be formed into dried particles is preferably first suspended or dissolved into a volatile liquid.

Examples of suitable liquids are water, ethanol, glycerol, methanol, DMSO, and mixtures thereof.

A small quantity of from about 0.1 picoliter to 1000 nanoliter of the liquid is deposited onto a substrate. Subsequently the deposited liquid that comprises the compound is subjected to a drying step. The drying results in the formation of particles that are separate and that do not form aggregates. This may be verified by for example microscopy techniques, e.g. SEM, AFM, optical microscopy, or light scattering methods.

It has been found that this drying to form separate particles is essential for their successful application in a variety of devices, especially biosensors, particularly those that are used for a quantitative assay method.

This drying in the form of separate particles may be obtained in variety of ways.

It is preferred that in step (b), prior to deposition, droplets of liquid are created which have an average volume of from 0.1*10⁻¹² to 100 *10⁻¹²1, preferably from 3*10⁻¹² to 50 *10⁻¹²1, even more preferred from 3 *10⁻¹² to 50 * 10⁻¹²1.

Preferably the concentration of compound in the liquid is such that each droplet comprises from 1 to 5, more preferred from 1 to 3, even more preferred 1 or 2 particles, most preferred 1 particle of the compound.

Generally such concentration is obtained by dilution of the liquid with a further liquid which may be the same or different, prior to the deposition step.

It will be appreciated that there may be a distribution in the amount of particles per droplet such that the average amount is between 1 and 5 and that the majority of the droplets do not contain more than 5 particles.

According to one embodiment, prior to deposition and drying, the droplets are introduced into liquid nitrogen. Preferably the droplets are shot into liquid nitrogen where they will freeze instantaneously.

Subsequently the droplets are subjected to drying, which is preferably in the form of freeze-drying. Alternatively the droplets are dried at a temperature around room temperature while printing the droplets on a surface. Such surface may be permeable or impermeable.

A very suitable technique to form the small droplets is by ink jet technology.

Alternative suitable technologies include electro spraying and spray drying technologies.

According to one embodiment, the droplets are directly deposited on a substrate such as a cartridge. Preferably the droplets are deposited with a frequency low enough to ensure drying in between.

A preferred deposition frequency is from low to medium in the preferred range of from about 0.1 to 10000 droplets per second, more preferred from 0.1 to 1000 droplets/second.

Generally the deposition process is expected to take about one second or preferably less than half a second. That way each particle is dried before another particle is formed. Preferably each subsequent droplet is deposited on a different place of the substrate to allow further drying of the previous droplet. Most preferred the distance between the places where subsequent droplets are deposited is from 0.5 to 100 micrometer.

In all embodiments of the invention it is highly preferred that the liquid further comprises a stabilizing agent. Such stabilizing agent may form a shell around the individual particles on drying, thus preventing direct contact between the particle surfaces in the dried state. Especially suitable stabilizing agents are those that dissolve readily in an aqueous composition upon redispersion of the dried particles.

Suitable stabilizing agent are preferably selected from the group comprising carbohydrates, polyelectrolytes, polymers, biomolecules such as proteins, surface active compositions or a mixture of any of these.

Preferably the concentration of such stabilizing agent in the liquid is chosen such that the particles are sufficiently coated upon drying. Furthermore, it is especially desired that the component does not interfere with the assay and the application after resuspension.

In step (b) the liquid comprising compound dissolved or dispersed therein, is deposited on a substrate. The substrate may be for example an impermeable glass surface, a micro cellulose membrane, a porous membrane, a plastic surface. It is preferred that the substrate is a permeable membrane.

The dried compounds are very suitable for use in sensor applications where they can e.g. be used to label molecules.

Therefore in a further aspect the invention relates to dried particles obtainable by the method of the invention. These particles differ from the ones that are prepared in prior art methods because they do not easily aggregate.

In another aspect the invention relates to a pharmaceutical composition comprising the dried particles.

Optionally such pharmaceutical composition further comprises a suitable pharmaceutical carrier.

The particles may be included in a medical device, especially a sensor, as a dry powder that is resuspended when the device is used. Therefore the invention also relates to a medical device comprising reservoirs comprising the dried particles. Such sensors will generally be small sensors comprising microreservoires of the particles. Upon adding a sample such as blood, blood serum, or urine the particles will redisperse without the formation of aggregates.

The reservoirs may be in any suitable format. In one embodiment the reservoirs are in the form of channels. In another embodiment the reservoir is in the form of a membrane. This membrane may be permeable or impermeable.

In a preferred embodiment, the device is a biosensor comprising a permeable membrane having immobilized thereon the dried particles according to the invention.

In another aspect of the invention the dried particles are applied as part of a contrast agent or precursor thereof for use in an imaging technique. Examples of imaging techniques are optical imaging, MRI imaging, ultrasound imaging and magnetic particle imaging. Before use the dried particles that comprise for example a label composition may be resuspended in a suitable carrier composition such as physiological salt solution.

A precursor of a contrast agent is for example part of a contrast agent or a composition that is activitated to act as a contrast agent in a later step, but which is currently masked.

We have found that the method according to the invention is especially suitable for the formation of dried magnetic particles. Such particles may be used to label molecules such as biomolecules, followed by read out of a magnetic signal by a magnetic sensor. Hence in a further aspect the invention relates to a medical device wherein the device is a biosensor and wherein the particles are dried magnetic particles.

In the context of a biosensor that detects the presence of magnetic particles (also called beads) in an assay, the particles are preferably magnetic beads, preferably stabilized with a coating (e.g. latex, polymethymetacryalte, polyeletrolytes, carbohydrate derivatives etc) and they have a preferred size range between 5-500 nm. Furthermore, these particles are preferably coated with a targeting ligand that may be an antibody or a derivative thereof, or a peptide or an organic molecule or an aptamer or a DNA or RNA molecule, or a protein, or any other molecule that shows a high affinity to a marker of interest. In the context of a biosoensor application a possible ligand is e.g. streptavidin, or biotin or protein.

Such a device may be used for the in vitro analysis of samples, which include blood, blood serum, urine, or other (processed) samples. The invention in another aspect relates to a method of in vitro analysis of a liquid sample comprising introducing the sample in a medical device as described above, wherein the dried particles are redispersed in the sample.

The redispersion preferably results in a homogeneous solution or dispersion of the particles in the sample.

A small shear force is optionally used to increase resuspension. This shear force is for example created when the dried powder is dissolved in a flowing liquid, for instance using water uptake through capillary forces in small channels in a plastic cartridge. The invention is illustrated by the following non-limiting example.

### Example

Colloidal suspensions of magnetic latex particles are used in many biomedical/sensor applications, where they can be used to label biomolecules. Eventually, the particles should be contained as a dry powder in a biosensor cartridge device. Upon adding the sample (e.g. blood), the particles should redisperse without forming aggregates. A typical commercially available aqueous solution of magnetic particles contains 10¹⁴ particles/L. Inkjet technique allows to make droplets with a volume of 3-10 · 10⁻¹² L per droplet at a rate up to about 10000 droplets per second using a single nozzle. The stock solution is diluted by a factor of 10 - 100 to obtain on average one particle per droplet. In this dilution step, suitable components to later coat the particles can be added.

Suppose a target of interest at a concentration of cₜ= 10 femtomol/l should be first extracted out of a 100 µl sample volume and subsequently detected using magnetic beads. We assume an association rate of kₒₙ=10⁶ l/(mol*s), a surface capture probe density of σ=1000 antibodies / µm², a radius of magnetic beads of R=100nm and an incubation time of τ=1000 seconds. After the incubation, we want 10000 beads to carry at least one target. Thus, N=10000 /(kₒₙ · cₜ·σ · τ · 4²)πR 10⁷ beads are required for the initial incubation step. Thus, 0.1 % of all beads capture a target. The beads are extracted using a magnet, washed, and resuspended in a smaller volume. The beads itself occupy a volume of ca. 100 picoliter. They can be resuspended in a volume of around one nanoliter. The target concentration (i.e. concration of beads carrying a target) is then 10000 in 10⁻⁹ L, corresponding to a nmol/L-pmol/L concentration range, which is measurable with a magnetic sensor. For an assay and detection inside a cartridge, 10⁷ beads are therefore required.

Using one nozzle with a rate of 10 000 droplets per second, it takes ca. 16 minutes to produce 10⁷ droplets.

## Claims

1. A method for making a dry solid of particles of a compound comprising the steps of
a) providing a volatile liquid comprising the compound dissolved or dispersed therein,
b) depositing a quantity of from 0.1-picoliter to 1000 nanoliter of the liquid onto a substrate
c) drying the liquid comprising the compound to produce a solid form of the compound
**characterized in that**
the concentration of the compound in the liquid that is deposited is such that the dried compound is in the form of separate particles.

2. A method according to claim 1 wherein in step (b) droplets of liquid are created which have an average volume of from 0.1* 10⁻¹² to 100 * 10⁻¹² 1, preferably from 3x10⁻¹² to 50 * 10⁻¹²1.

3. A method according to claim 2 wherein prior to deposition and drying, the droplets are introduced into liquid nitrogen.

4. A method according to claim 1 wherein the drying is done by freeze-drying technology.

5. A method according to claim 2 wherein the droplets are created by ink jet technology.

6. A method according to claim 2 wherein the droplets are deposited on a substrate with a low frequency from 0.1 to 10000 droplets per second.

7. A method according to claim 1 wherein the liquid further comprises a stabilizing agent.

8. A method according to claim 7 wherein the stabilizing agent is selected from the group comprising carbohydrates, polyelectrolytes, polymers, biomolecules such as proteins, surface active compositions or a mixture of any of these.

9. A method according to claim 1 wherein the compound is selected from the group comprising biomolecules, magnetic particles, quantum dots, gold or silver nanoparticles .

10. Dried particles obtainable by the method according to any of claims 1-9.

11. Pharmaceutical composition comprising dried particles according to claim 10.

12. Contrast agent or precursor thereof comprising dried particles according to claim 10.

13. A medical device comprising reservoirs comprising the dried particles according to claim 10.

14. A medical device according to claim 13 wherein the device is a biosensor and wherein the particles are dried magnetic particles.

15. A method of in vitro analysis of a liquid sample comprising introducing the sample in a medical device according to claim 13, wherein the dried particles are redispersed in the sample.
